(19) Europäisches Patentamt
European Patent Office
Office européen des brevets

(11) Veröffentlichungsnummer: **0 473 041 A1**

(12) **EUROPÄISCHE PATENTANMELDUNG**

(21) Anmeldenummer: **91113881.6**

(51) Int. Cl.[5]: **C07D 307/80**, A01N 43/12

(22) Anmeldetag: **20.08.91**

(30) Priorität: **31.08.90 DE 4027572**

(43) Veröffentlichungstag der Anmeldung:
**04.03.92 Patentblatt 92/10**

(84) Benannte Vertragsstaaten:
**BE CH DE DK ES FR GB IT LI NL**

(71) Anmelder: **BASF Aktiengesellschaft**
**Carl-Bosch-Strasse 38**
**W-6700 Ludwigshafen(DE)**

(72) Erfinder: **Mueller, Stefan, Dr.**
**Closweg 7**
**W-6720 Speyer(DE)**
Erfinder: **Wolf, Bernd, Dr.**
**Halbergstrasse 4**
**W-6701 Fussgoenheim(DE)**
Erfinder: **Theobald, Hans, Dr.**
**Oueichstrasse 6**
**W-6703 Limburgerhof(DE)**
Erfinder: **Harreus, Albrecht, Dr.**
**Teichgasse 13**
**W-6700 Ludwigshafen(DE)**
Erfinder: **Kardorf, Uwe, Dr.**
**D 3,4**
**W-6800 Mannheim 1(DE)**
Erfinder: **Kuenast, Christoph, Dr.**
**Salierstrasse 2**
**W-6701 Otterstadt(DE)**

(54) **2,3-Dihydrobenzofurylmethylester, ihre Herstellung und sie enthaltende Mittel zur Bekämpfung von Schädlingen.**

(57) 2,3-Dihydrobenzofurylmethylester der allgemeinen Formel I,

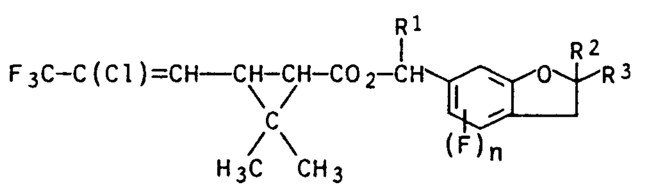

I

in der die Substituenten und der Index folgende Bedeutung haben:

- $R^1$ Wasserstoff, $C_1$-$C_4$-Alkyl, $C_2$-$C_4$-Alkenyl, $C_2$-$C_4$-Alkinyl oder Cyano;
- $R^2$ und $R^3$ unabhängig voneinander Wasserstoff oder $C_1$-$C_4$-Alkyl und
- n 0 oder 1,

Verfahren zu Ihrer Herstellung und sie enthaltende Mittel zur Bekämpfung von Schädlingen.

EP 0 473 041 A1

Die vorliegende Erfindung betrifft 2,3-Dihydrobenzofurylmethylester der allgemeinen Formel I,

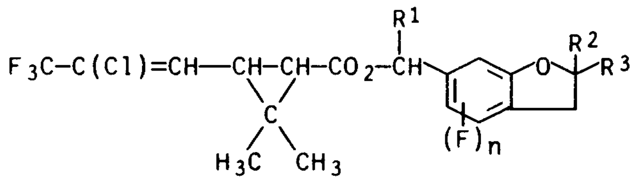

I

in der die Substituenten und der Index folgende Bedeutung haben:

- $R^1$ Wasserstoff, $C_1$-$C_4$-Alkyl, $C_2$-$C_4$-Alkenyl, $C_2$-$C_4$-Alkinyl oder Cyano;
- $R^2$ und $R^3$ unabhängig voneinander Wasserstoff oder $C_1$-$C_4$-Alkyl und
- n 0 oder 1.

Außerdem betrifft die Erfindung Verfahren zur Herstellung der Verbindungen I, sie enthaltende Mittel und Verfahren zu ihrer Verwendung zur Bekämpfung von Schädlingen.

In der DE-A 21 08 932 werden Chrysanthemumsäure-2,2-dimethyl-2,3-dihydrobenzofuryl-6-methylester mit insektizider Wirkung beschrieben. Außerdem sind aus der EP-A 23 637 2,3-Dihydro-benzofurylmethylester bekannt, deren allgemeine Formel die eingangs beschriebenen 2,3-Dihydrobenzofurylmethylester der allgemeinen Formel I umfaßt.

Aufgabe der vorliegenden Erfindung waren neue Verbindungen mit verbesserter insektizider und akarizider Wirkung.

Demgemäß wurden die eingangs definierten 2,3-Dihydrobenzofurylmethylester I gefunden. Außerdem wurden Verfahren zur Herstellung dieser 2,3-Dihydrobenzofurylmethylester, sie enthaltende Mittel und Verfahren zur Verwendung dieser Verbindungen als Insektizide und Akarizide gefunden.

Die 2,3-Dihydrobenzofurylmethylester I sind auf verschiedenen Wegen zugänglich. Besonders vorteilhaft erhält man sie nach einem der im nachfolgenden beschriebenen Verfahren A und B.

Verfahren A

Man erhält die Verbindungen I beispielsweise dadurch, daß man ein 2,3-Dihydrobenzofurylmethylderivat der allgemeinen Formel II in an sich bekannter Weise mit einer Carbonsäure der allgemeinen Formel IIIa oder deren Derivaten verestert.

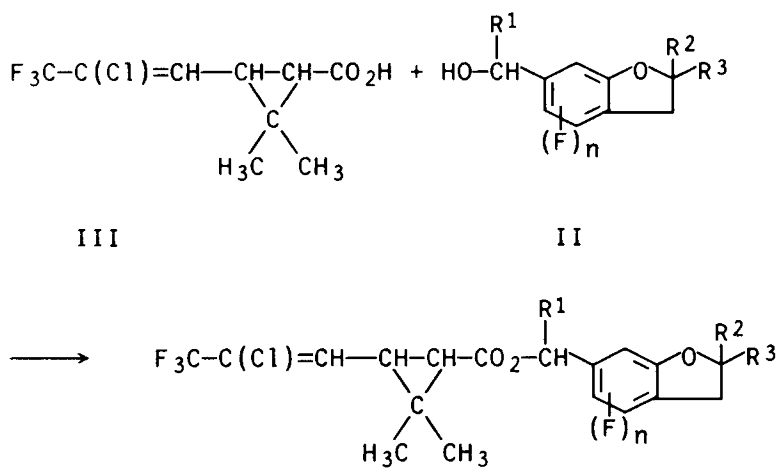

III

II

I

Diese Umsetzung kann nach den üblichen, aus der Literatur bekannten Veresterungsmethoden durchgeführt werden.

1. Sofern man bei dieser Umsetzung von einer Carbonsäure und einem Alkohol ausgeht, arbeitet man üblicherweise bei Temperaturen von -10 °C bis 120 °C, vorzugsweise 10 °C bis 40 °C in einem inerten organischen Lösungsmittel in Gegenwart eines sauren Katalysators (Literatur Houben-Weyl, Bd. VIII, S.

516 f.).

Für diese Veresterungsmethode eignen sich besonders aprotische apolare Lösungsmittel wie aliphatische und aromatische, gegebenenfalls chlorierte Kohlenwasserstoffe, z.B. Petrolether, Benzol, Toluol, Xylol, Benzin, Dichlormethan, Chloroform, Tetrachlormethan, 1,2-Dichlorethan, Chlorbenzol; Ether wie Diethyl- und Di-n-butylether, Methyl-tert.-butylether, Tetrahydrofuran, Dioxan; Ketone, beispielsweise Aceton, Methylethylketon, Methylisopropylketon; ferner Nitrile wie Acetonitril und Propionitril sowie entsprechende Gemische. Als saure Katalysatoren finden beispielsweise Schwefelsäure, Halogenwasserstoffe, Sulfonsäuren und saure Ionenaustauscher bzw. Polymere Verwendung.

Der Katalysator wird im allgemeinen in Mengen von 0,001 mol-Äq. bis 10 mol-Äq., vorzugsweise 0,01 mol-Äq. bis 0,1 mol-Äq. bezogen auf die Carbonsäure IIIa eingesetzt.

Außerdem kann das Reaktionsgleichgewicht im gewünschten Sinne verschoben werden, indem man dem Reaktionsgemisch das bei der Umsetzung gebildete Wasser oder den Ester entzieht, z.B. durch azeotrope Destillation oder durch Bindung des Wassers in Schwefel- oder Halogenwasserstoffsäure oder Bindung des Wassers an Molekularsiebe.

Im allgemeinen werden Alkohol und Carbonsäure in äquimolaren Mengen miteinander umgesetzt. Es kann für die Ausbeute vorteilhaft sein, den Alkohol in einem Über- oder Unterschuß bezogen auf die Carbonsäure einzusetzen.

2. Sofern man bei dieser Umsetzung von einer aktivierten Carbonsäure und einem Alkohol ausgeht, arbeitet man üblicherweise bei Temperaturen von 0 °C bis 100 °C, vorzugsweise 15 °C bis 25 °C in einem inerten organischen Lösungsmittel in Gegenwart einer Base (Houben-Weyl, Bd. E5, S. 691 f.).

Als aktivierte Carbonsäurederivate dienen beispielsweise Anhydride, Halogenide wie Chloride und Bromide oder Imidazolide.

Für diese Veresterungsmethode eignen sich besonders die vorstehend genannten aprotisch apolaren Lösungsmittel.

Als Basen finden besonders aliphatische, aromatische und heterocyclische Amine wie Dimethylamin, Triethylamin, Piperidin, Dimethylanilin, Dimethylbenzylamin, Pyridin und 2-Picolin Verwendung.

Die Base wird im allgemeinen in einem Überschuß bis zu 5,0, vorzugsweise bis zu 2,0 mol-Äq., insbesondere von 1,1 mol-Äq. bis 1,35 mol-Äq. bezogen auf das Carbonsäurederivat eingesetzt.

Die vorstehend genannten Basen können auch direkt als Lösungsmittel verwendet werden.

Im allgemeinen werden der Alkohol und das Carbonsäurederivat in äquimolaren Mengen miteinander umgesetzt. Es kann für die Ausbeute vorteilhaft sein, den Alkohol in einem Über- oder Unterschuß bezogen auf das Carbonsäurederivat einzusetzen.

3. Neben den vorstehend beschriebenen Möglichkeiten kann man zur Veresterung bekanntermaßen auch anstelle des Alkohols von einem entsprechenden aktivierten Derivat ausgehen welches mit dem Salz einer Carbonsäure verestert wird.

Geeignete, für diese Art der Veresterung aktivierte Alkoholderivate sind beispielsweise die entsprechenden Halogenide, insbesondere Chloride und Bromide sowie die Sulfonsäureester wie Toluolsulfonsäureester und Benzolsulfonsäureester.

Als Salze der Carbonsäuren finden Alkalimetall-, Erdalkalimetall-, Silber- und Bleisalze Verwendung.

Bei dieser Verfahrensweise arbeitet man üblicherweise bei Temperaturen von -10 °C bis 150 °C, vorzugsweise 80 °C bis 130 °C in einem polaren inerten Lösungsmittel in Gegenwart einer der im allgemeinen und im besonderen bei der unter 2. beschriebenen Verfahrensweise genannten Basen (Houben-Weyl, Bd. VIII, S. 541 f. und Bd E5, S. 684 f).

Für diese Umsetzung eignen sich Lösungsmittel wie Wasser, Alkohole wie insbesondere Methanol, Ethanol und Isopropanol, Dimethylformamid und Dimethylsulfoxid.

Die Herstellung der Carbonsäuresalze erfolgt durch Umsetzung der Carbonsäuren mit den entsprechenden Metallhydroxiden, -carbonaten und -hydrogencarbonaten in den vorstehend genannten Lösungsmitteln. Bei geeigneter Wahl der Reaktionsparameter kann die Umsetzung auch im 'Eintopfverfahren', d.h. ohne Isolierung der Carbonsäuresalze durchgeführt werden.

4. Daneben können die Ester des Typs I auch durch Umesterung erhalten werden. Hierzu werden Niederalkylester (vorzugsweise Methyl- und Ethylester) der Carbonsäure IIIa in an sich bekannter Weise (Houben-Weyl, Bd. E5, S. 702 f.; GB-A 2 005 269) in einem inerten, aprotisch apolaren Lösungsmittel wie im allgemeinen und im besonderen bei Verfahren A1. aufgeführt, mit dem entsprechenden Alkohol II (Z = OH) gegebenenfalls in Gegenwart eines Katalysators umgesetzt. Als Katalysatoren kommen hierbei anorganische Säuren wie Schwefelsäure und Halogenwasserstoffsäure sowie Lewis-Säuren wie Bortrifluorid, Aluminiumtrichlorid, Titantetrachlorid und Tetraalkoxy-titanate zur Anwendung.

Im allgemeinen werden das Alkoholderivat und die Carbonsäure in äquimolaren Mengen miteinander umgesetzt. Es kann für die Ausbeute vorteilhaft sein, das Alkoholderivat in einem Über- oder Unterschuß

bezogen auf die Carbonsäure einzusetzen.

Verfahren B

Verbindungen I, in denen $R^1$ Cyano bedeutet, lassen sich neben den vorstehend geschilderten Verfahren auch dadurch herstellen daß man einen 2,3-Dihydrobenzofurylaldehyd der allgemeinen Formel IV in an sich bekannter Weise in Gegenwart eines Alkalimetall- oder eines Erdalkalimetallcyanids mit dem Halogenid einer Carbonsäure der allgemeinen Formel IIIb umsetzt.

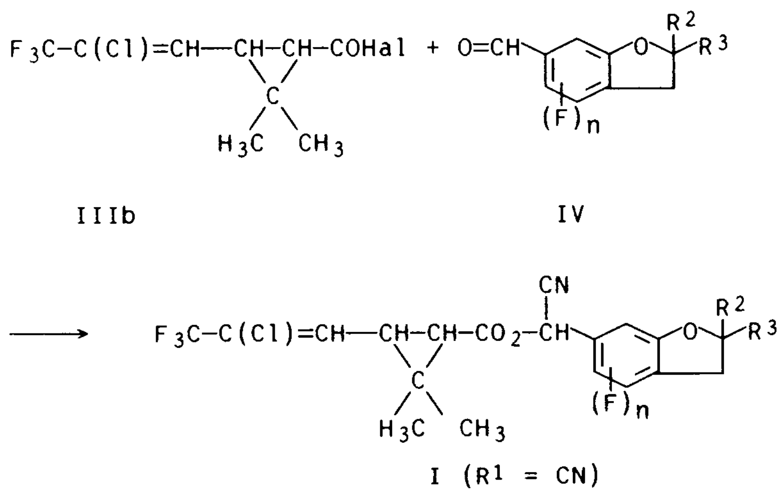

IIIb IV

I ($R^1$ = CN)

Hal in der Formel IIIb bedeutet ein Halogenatom wie Fluor, Chlor, Brom und Jod, insbesondere Chlor und Brom.

Diese Umsetzung wird üblicherweise bei Temperaturen von 0°C bis 100°C, vorzugsweise 15°C bis 25°C in einem inerten organischen Lösungsmittel oder in einem 2-Phasen-System unter Zusatz von Wasser durchgeführt.

Besonders geeignet sind aprotische apolare Lösungsmittel wie aliphatische und aromatische, gegebenenfalls halogenierte Kohlenwasserstoffe.

Sofern man die Umsetzung in einem Zweiphasensystem durchführt, kann es von Vorteil sein, der Mischung einen Phasentransfer-Katalysator zuzusetzen (Dehmlow et al., Phase Transfer Catalysis, Verlag Chemie, Weinheim (1983)). Geeignete Phasentransfer-Katalysatoren sind beispielsweise quarternäre Ammonium- und Phosphonium-Salze, insbesondere Tetrabutylammonium-chlorid. Die Menge des zugesetzten Katalysators beträgt im allgemeinen 0,2 bis 20 mol-%, vorzugsweise 1 bis 5 mol-%, bezogen auf die Verbindung IV.

Als Alkalimetall- und Erdalkalimetallcyanide finden die Cyanide des Lithiums, des Natriums, des Kaliums, des Cäsiums, des Calziums und des Bariums Verwendung.

Das Cyanid wird im allgemeinen in Mengen von 1,0 mol-Äq. bis 5,0 mol-Äq., vorzugsweise 1,05 mol-Äq. bis 1,2 mol-Äq. bezogen auf den Aldehyd IV eingesetzt.

Im allgemeinen werden Aldehyd und Carbonsäurehalogenid in äquimolaren Mengen miteinander umgesetzt. Es kann für die Ausbeute vorteilhaft sein, den Aldehyd in einem Unterschuß bezogen auf das Carbonsäurehalogenid einzusetzen.

Die für die Herstellung der Verbindungen I benötigten Ausgangsstoffe sind in der Literatur bekannt (Carbonsäuren und deren Derivate siehe EP-A 3 336; 2,3-Dihydrobenzofurylmethylalkohole siehe EP-A 23 637) oder können gemäß der zitierten Literatur hergestellt werden.

Besonders vorteilhaft erhält man die Alkohole der allgemeinen Formel II, indem man ein 2,3-Dihydrobenzofurylhalogenid der allgemeinen Formel V in an sich bekannter Weise metalliert, anschließend formyliert und den so erhaltenen Benzaldehyd der allgemeinen Formel IV entweder reduziert oder mit einer metallorganischen Verbindung $MR^1$ umsetzt.

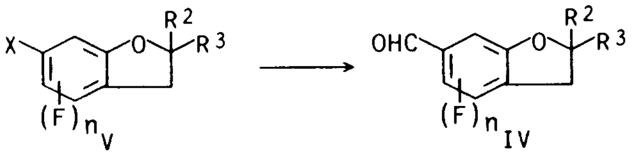

V IV

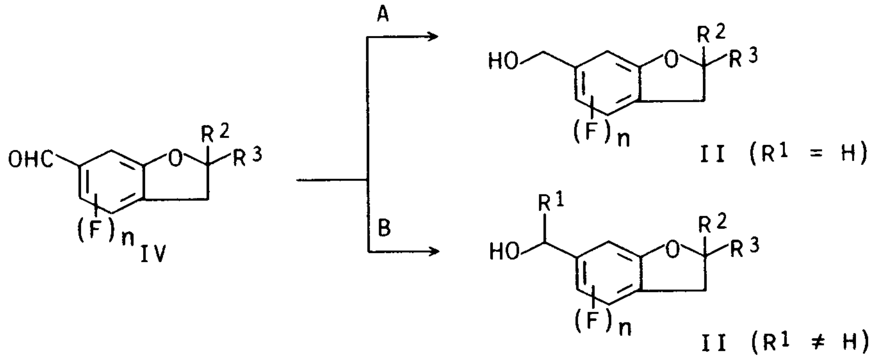


IV II ($R^1$ = H) II ($R^1$ ≠ H)

X in der Formel V bedeutet Chlor, Brom oder Jod, insbesondere Brom.

Für die Umsetzung von V zu IV wird als Metallierungs-Reagens üblicherweise Magnesium in Form von Spänen eingesetzt (Grignard-Reagens). Es ist jedoch auch denkbar, die Verbindung V durch Umsetzung mit einem entsprechenden Alkalimetallorganyl wie t.-Butyl-lithium oder Phenyl-lithium in ein entsprechendes Metallorganyl zu überführen. Das auf diesem Wege hergestellte Metallorganyl der Verbindung V wird anschließend, üblicherweise in situ, mit einem Formylierungsmittel wie vorzugsweise N-Formylpiperidin oder Dimethylformamid zum Benzaldehyd IV umgesetzt (Houben-Weyl, Bd. E3, S. 116 f.).

Die weitere Umsetzung von IV zu II erfolgt in an sich bekannter Weise entweder mit einem gebräuchlichen Reduktionsmittel wie Wasserstoff bzw. Alkalimetall- oder Erdalkalimetallhydriden zu den primären Alkoholen ($R^1$ = H) oder durch Umsetzung mit einer metallorganischen Verbindung $MR^1$, in der M für ein Alkalimetall, ein Erdalkalimetall oder ein Übergangsmetall steht und $R^1$ nicht Wasserstoff bedeutet (Gattermann-Wieland, Walter de Gruyter Verlag, Berlin (1982)).

Die Verbindungen der Formel I können nach den beschriebenen Verfahren sowohl in Form von einheitlichen Isomeren, Enantiomeren und Diastereomeren als auch in Form von Gemischen der Strukturisomere erhalten werden und als solche als Wirkstoffe angewandt werden. Die Gemische der Strukturisomeren (Enantiomeren, Diastereomeren) können in üblicher Weise in ihre sterisch einheitlichen Bestandteile aufgetrennt werden; ihre biologische Wirkung ist in Einzelfällen von ihrer sterischen Konfiguration abhängig. Bevorzugt kommen solche Isomere zur Anwendung, in denen die Substituenten des Cyclopropanrings cis zueinander angeordnet sind.

Im Hinblick auf die bestimmungsgemäße Verwendung der Verbindungen I in Schädlingsbekämpfungsmitteln kommen als Substituenten folgende Reste in Betracht:

$R^1$ Wasserstoff;
$C_1$-$C_4$-Alkyl wie Methyl, Ethyl, Propyl, 1-Methylethyl, Butyl, 1-Methylpropyl, 2-Methylpropyl und 1,1-Dimethylethyl, vorzugsweise Methyl und 1-Methylethyl;
$C_2$-$C_4$-Alkenyl wie Ethenyl, 1-Propenyl, 2-Propenyl, 1-Methyl ethenyl, 1-Butenyl, 2-Butenyl, 3-Butenyl, 1-Methyl-1-propenyl, 2-Methyl-1-propenyl, 1-Methyl-2-propenyl und 2-Methyl-2-propenyl, vorzugsweise Ethenyl;
$C_2$-$C_4$-Alkinyl wie Ethinyl, 1-Propinyl, 2-Propinyl, 1-Butinyl, 2-Butinyl, 3-Butinyl und 1-Methyl-2-propinyl, vorzugsweise Ethinyl;
oder Cyano;

$R^2$ und $R^3$ unabhängig voneinander Wasserstoff oder $C_1$-$C_4$-Alkyl wie vorstehend bei $R^1$ genannt, vorzugsweise Methyl, Ethyl und Propyl und

n 0 oder 1.

Besonders bevorzugt sind 2,3-Dihydrobenzofurylmethylester der allgemeinen Formel I in denen

$R^1$ Wasserstoff,
$C_1$-$C_4$-Alkyl wie vorstehend im allgemeinen und im besonderen genannt, $C_2$-$C_4$-Alkinyl wie vorstehend im allgemeinen und im besonderen genannt, oder Cyano,

$R^2$ und $R^3$ unabhängig voneinander Wasserstoff oder $C_1$-$C_4$-Alkyl wie vorstehend im allgemeinen und im besonderen genannt, und n = 0 bedeuten.

Außerdem werden 2,3-Dihydrobenzofurylmethylester der allgemeinen Formel I bevorzugt, in denen die Reste folgende Bedeutung haben:

$R^1$ Wasserstoff, $C_1$-$C_4$-Alkyl wie vorstehend im allgemeinen und im besonderen genannt, $C_2$-$C_4$-Alkenyl wie vorstehend im allgemeinen und im besonderen genannt, $C_2$-$C_4$-Alkinyl wie vorstehend im allgemeinen und im besonderen genannt oder Cyano,

$R^2$ und $R^3$ unabhängig voneinander $C_1$-$C_4$-Alkyl wie vorstehend im allgemeinen und im besonderen genannt und $n = 0$.

Beispiele für die insbesondere bevorzugten 2,3-Dihydrobenzofurylmethylester der allgemeinen Formeln IA und IB sind in der folgenden Tabelle aufgeführt.

Tabelle

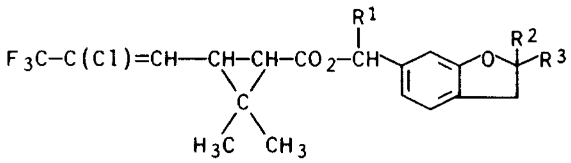

IA

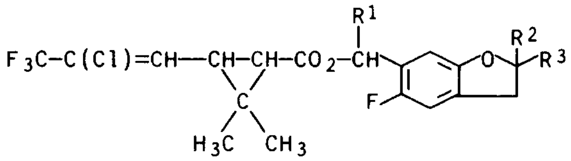

IB

| $R^1$ | $R^2$ | $R^3$ |
|---|---|---|
| H | $CH_3$ | $CH_3$ |
| $CH(CH_3)_2$ | $CH_3$ | $CH_3$ |
| C≡CH | $CH_3$ | $CH_3$ |
| CN | $CH_3$ | $CH_3$ |
| H | $CH_3$ | $CH_2CH_3$ |
| $CH(CH_3)_2$ | $CH_3$ | $CH_2CH_3$ |
| C≡CH | $CH_3$ | $CH_2CH_3$ |
| CN | $CH_3$ | $CH_2CH_3$ |
| H | $CH_2CH_3$ | $CH_2CH_3$ |
| $CH(CH_3)_2$ | $CH_2CH_3$ | $CH_2CH_3$ |
| C≡CH | $CH_2CH_3$ | $CH_2CH_3$ |
| CN | $CH_2CH_3$ | $CH_2CH_3$ |
| H | $CH_3$ | $CH_2CH_2CH_3$ |
| $CH(CH_3)_2$ | $CH_3$ | $CH_2CH_2CH_3$ |
| C≡CH | $CH_3$ | $CH_2CH_2CH_3$ |
| CN | $CH_3$ | $CH_2CH_2CH_3$ |
| H | $CH_3$ | $CH(CH_3)_2$ |
| $CH(CH_3)_2$ | $CH_3$ | $CH(CH_3)_2$ |
| C≡CH | $CH_3$ | $CH(CH_3)_2$ |
| CN | $CH_3$ | $CH(CH_3)_2$ |
| H | $CH_3$ | $(CH_2)_3CH_3$ |
| $CH(CH_3)_2$ | $CH_3$ | $(CH_2)_3CH_3$ |
| C≡CH | $CH_3$ | $(CH_2)_3CH_3$ |
| CN | $CH_3$ | $(CH_2)_3CH_3$ |
| H | $CH_3$ | $CH(CH_3)CH_2CH_3$ |
| $CH(CH_3)_2$ | $CH_3$ | $CH(CH_3)CH_2CH_3$ |
| C≡CH | $CH_3$ | $CH(CH_3)CH_2CH_3$ |
| CN | $CH_3$ | $CH(CH_3)CH_2CH_3$ |
| H | $CH_3$ | $C(CH_3)_3$ |
| $CH(CH_3)_2$ | $CH_3$ | $C(CH_3)_3$ |

Tabelle (Fortsetzung)

| $R^1$ | $R^2$ | $R^3$ |
|---|---|---|
| C≡CH | $CH_3$ | $C(CH_3)_3$ |
| CN | $CH_3$ | $C(CH_3)_3$ |
| H | H | H |
| $CH(CH_3)_2$ | H | H |
| C≡CH | H | H |
| CN | H | H |

Die Verbindungen der Formel I sind geeignet, Schädlinge aus der Klasse der Insekten, Spinntiere und Nematoden wirksam zu bekämpfen. Sie können im Pflanzenschutz sowie auf dem Hygiene-, Vorratsschutz- und Veterinärsektor als Schädlingsbekämpfungsmittel eingesetzt werden.

Zu den schädlichen Insekten gehören aus der Ordnung der Schmetterlinge (Lepidoptera) beispielsweise Agrotis ypsilon, Agrotis segetum, Alabama argillacea, Anticarsia gemmatalis, Argyresthia conjugella, Autographa gamma, Bupalus piniarius, Cacoecia murinana, Capua reticulana, Cheimatobia brumata, Choristoneura fumiferana, Choristoneura occidentalis, Cirphis unipuncta, Cydia pomonella, Dendrolimus pini, Diaphania nitidalis, Diatraea grndiosella, Earias insulana, Elasmopalpus lignosellus, Eupoecilia ambiguella, Evetria bouliana, Feltia subterranea, Galleria mellonella, Grapholita funebrana, Grapholita molesta, Heliothis armigera, Heliothis virescens, Heliothis zea, Hellula undalis, Hibernia defoliaria, Hyphantria cunea, Hyponomeuta malinellus, Keifferia lycopersicella, Lambdina fiscellaria, Laphygma exigua, Leucoptera coffeella, Leucoptera scitella, Lithocolletis blancardella, Lobesia botrana, Loxostege sticticalis, Lymantria dispar, Lymantria monacha, Lyonetia clerkella, Malacosoma neustria, Mamestra brassicae, Orgyia pseudotsugata, Ostrinia nubilalis, Panolis flamea, Pectinophora gossypiella, Peridroma saucia, Phalera bucephala, Phthorimaea operculella, Phyllocnistis citrella, Pieris brassicae, Plathypena scarbra, Plutella xylostella, Pseudoplusia includens, Phyacionia frustrana, Scrobipalpula absoluta, Sitotroga cerelella, Sparganothis pilleriana, Spodoptera frugiperda, Spodoptera littoralis, Spodoptera litura, Thaumatopoea pityocampa, Tortrix viridana, Trichoplusia ni, Zeiraphera canadensis.

Aus der Ordnung der Käfer (Coleoptera) beispielsweise Agrilus sinuatus, Agriotes lineatus, Agriotes obscurus, Amphimallus solstitialis, Anisandrus dispar, Anthonomus grandis, Anthonomus pomorum, Atomaria linearis, Blastophagus piniperda, Blitophaga undata, Bruchus rufimanus, Bruchus pisorum, Bruchus lentis, Byctiscus betulae, Cassida nebulosa, Cerotoma trifurcata, Ceuthorrhynchus assimilis, Ceuthorrynchus napi, Chaetocnema tibialis, Conoderus vespertinus, Crioceris asparagi, Diabrotica longicornis, Diabrotica 12-punctata, Diabrotica virgifera, Epilachna varivestis, Epitrix hirtipennis, Eutinobothrus brasiliensis, Hylobius abietis, Hypera brunneipennis, Hypera postica, Ips typographus, Lema bilineata, Lema melanopus, Leptinotarsa decemlineata, Limonius californicus, Lissorhoptrus oryzophilus, Melanotus communis, Meligethes aeneus, Melolontha hippocastani, Melolontha melolontha, Onlema oryzae, Ortiorrhynchus sulcatus, Otiorrhynchus ovatus, Phaedon cochleariae, Phyllotreta chrysocephala, Phyllophaga sp., Phyllopertha horticola, Phyllotreta nemorum, Phyllotreta striolata, Popillia japonica, Sitona lineatus, Sitophilus granaria.

Aus der Ordnung der Zweiflügler (Diptera) beispielsweise Aedes aegypti, Aedes vexans, Anastrepha ludens, Anopheles maculipennis, Ceratitis capitata, Chrysomya bezziana, Chrysomya hominivorax, Chrysomya macellaria, Contarinia sorghicola, Cordylobia anthropophaga, Culex pipiens, Dacus cucurbitae, Dacus oleae, Dasineura brassicae, Fannia canicularis, Gasterophilus intestinalis, Glossia morsitans, Haematobia irritans, Haplodiplosis equestris, Hylemyia platura, Hypoderma lineata, Liriomyza sativae, Liriomyza trifolii, Lucilia caprina, Lucilia cuprina, Lucilia sericata, Lycoria pectoralis, Mayetiola destructor, Musca domestica, Muscina stabulans, Oestrus ovis, Oscinella frit, Pegomya hysocyami, Phorbia antiqua, Phorbia brassicae, Phorbia coarctata, Rhagoletis cerasi, Rhagoletis pomonella, Tabanus bovinus, Tipula oleracea, Tipula paludosa.

Aus der Ordnung der Thripse (Thysanoptera) beispielsweise Frankliniella fusca, Frankliniella occidentalis, Frankliniella tritici, Scirtothrips citri, Thrips oryzae, Thrips palmi, Thrips tabaci.

Aus der Ordnung der Hautflügler (Hymenoptera) beispielsweise Athalia rosae, Atta cephalotes, Atta sexdens, Atta texana, Hoplocampa minuta, Hoplocampa testudinea, Monomorium pharaonis, Solenopsis geminata, Solenopsis invicta.

Aus der Ordnung der Wanzen (Heteroptera) beispielsweise Acrosternum hilare, Blissus leucopterus, Cyrtopeltis notatus, Dysdercus cingulatus, Dysdercus intermedius, Eurygaster integriceps, Euchistus impictiventris, Leptoglossus phyllopus, Lygus lineolaris, Lygus pratensis, Nezara viridula, Piesma quadrata, Solubea insularis, Thyanta perditor.

Aus der Ordnung der Pflanzensauger (Homoptera) beispielsweise Acyrthosiphon onobrychis, Adelges laricis, Aphidula nasturtii, Aphis fabae, Aphis pomi, Aphis sambuci, Brachycaudus cardui, Brevicoryne brassicae, Cerosipha gossypii, Dreyfusia nordmannianae, Dreyfusia piceae, Dyasphis radicola, Dysaulacorthum pseudosolani, Empoasca fabae, Macrosiphum avenae, Macrosiphum euphorbiae, Macrosiphon rosae, Megoura viciae, Metopolophium dirhodum, Myzodes persicae, Myzus cerasi, Nilaparvata lugens, Pemphigus bursarius, Perkinsiella saccharicida, Phorodon humuli, Psylla mali, Psylla piri, Rhopalomyzus ascalonicus, Rhopalosiphum maidis, Sappaphis mala, Sappaphis mali, Schizaphis graminum, Schizoneura lanuginosa, Trialeurodes vaporariorum, viteus vitifolii.

Aus der Ordnung der Termiten (Isoptera) beispielsweise Calotermes flavicollis, Leucotermes flavipes, Reticulitermes lucifugus, Termes natalensis.

Aus der Ordnung der Geradflügler (Orthoptera) beispielsweise Acheta domestica, Blatta orientalis, Blattella germanica, Forficula auricularia, Gryllotalpa gryllotalpa, Locusta migratoria, Melanoplus birittatus, Melanoplus femur-rubrum, Melanoplus mexicanus, Melanoplus sanguinipes, Melanoplus spretus, Nomadacris septemfasciata, Periplaneta americana, Schistocerca americana, Schistocerca peregrina, Stauronotus maroccanus, Tachycines asynamorus.

Aus der Klasse der Arachnoidea beispielsweise Spinnentiere (Acarina) wie Amblyomma americanum, Amglyomma variegatum, Argas persicus, Boophilus annulatus, Boophilus decoloratus, Boophilus microplus, Brevipalpus phoenicis, Bryobia praetiosa, Dermacentor silvarum, Eotetranychus carpini, Eriophyes sheldoni, Hyalomma truncatum, Ixodes ricinus, Ixodes rubicundus, Ornithodorus moubata, Otobins megnini, Paratetranychus pilosus, Permanyssus gallinae, Phyllocaptrata oleivora, Polyphagotarsonemus latus, Psoroptes ovis, Rhipicephalus appendiculatus, Rhipicephalus evertsi, Saccoptes scabiei, Tetranychus cinnabarinus, Tetranychus kanzawai, Tetranychus pacificus, Tetranychus telarius, Tetranychus urticae.

Aus der Klasse der Nematoden beispielsweise Wurzelgallennematoden, z.B. Meloidogyne hapla, Meloidogyne incognita, Meloidogyne javanica, Zysten bildende Nematoden, z.B. Globodera rostochiensis, Heterodera avenae, Heterodera glycinae, Heterodera schatii, Hetrodera triflolii, Stock- und Blattälchen, z.B. Belonolaimus longicaudatus, Ditylenchus destructor, Ditylenchus dipsaci, Heliocotylenchus multicinctus, Longidorus elongatus, Radopholus similis, Rotylenchus robustus, Trichodorus primitivus, Tylenchorhynchus claytoni, Tylenchorhynchus dubius, Pratylenchus neglectus, Pratylenchus penetrans, Pratylenchus curvitatus, Pratylenchus goodeyi.

Die Wirkstoffe können als solche, in Form ihrer Formulierungen oder den daraus bereiteten Anwendungsformen, z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, Methanol, Ethanol, Propanol, Butanol, Chloroform, Tetrachlorkohlenstoff, Cyclohexanol, Cyclohexanon, Chlorbenzol, Isophoron, stark polare Lösungsmittel, z.B. Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern (Spritzpulver, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermitttel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Dibutylnaphthalinsulfonsäure, Alkylarylsulfonate, Alkylsulfate, Alkylsulfonate, Fettalkoholsulfate und Fettsäuren sowie deren Alkali- und Erdalkalisalze, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphtalinsulfonsäure mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin-Sulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Die Formulierungen enthalten im allgemeinen zwischen 0,01 und 95 Gew.%, vorzugsweise zwischen 0,1 und 90 Gew.% des Wirkstoffs. Die Wirkstoffe werden dabei in einer Reinheit von 90 % bis 100 %, vorzugsweise 95 % bis 100 % (nach NMR-Spektrum) eingesetzt.

Beispiele für Formulierungen sind:

I. 5 Gew.-Teile der Verbindung Nr. 1.001 werden mit 95 Gew.-Teilen feinteiligem Kaolin innig vermischt. Man erhält auf diese Weise ein Stäubemittel, das 5 Gew.% des Wirkstoffs enthält.

II. 30 Gew.-Teile der Verbindung Nr. 1.002 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit (Wirkstoffgehalt 23 Gew.%).

III. 10 Gew.-Teile der Verbindung Nr. 1.001 werden in einer Mischung gelöst, die aus 90 Gew.-Teilen Xylol, 6 Gew.-Teilen des Anlagerungsproduktes von 8 bis 10 Mol Ethylenoxid an 1 Mol Ölsäure-N-monoethanolamid, 2 Gew.-Teilen Calciumsalz der Dodecylbenzolsulfonsäure und 2 Gew.-Teilen des Anlagerungsproduktes von 40 Mol Ethylenoxid an 1 Mol Ricinusöl besteht (Wirkstoffgehalt 9 Gew.%).

IV. 20 Gew.-Teile der Verbindung Nr. 1.010 werden in einer Mischung gelöst, die aus 60 Gew.-Teilen Cyclohexanon, 30 Gew.-Teilen Isobutanol, 5 Gew.-Teilen des Anlagerungsproduktes von 7 Mol Ethylenoxid an 1 Mol Isooctylphenol und 5 Gew.-Teilen des Anlagerungsproduktes von 40 Mol Ethylenoxid an 1 Mol Ricinusöl besteht (Wirkstoffgehalt 16 Gew.%).

V. 80 Gew.-Teile der Verbindung Nr. 1.002 werden mit 3 Gew.-Teilen des Natriumsalzes der Diisobutylnaphthalin-alpha-sulfonsäure, 10 Gew.-Teilen des Natriumsalzes einer Ligninsulfonsäure aus einer Sulfit-Ablauge und 7 Gew.-Teilen pulverförmigem Kieselsäuregel gut vermischt und in einer Hammermühle vermahlen (Wirkstoffgehalt 80 Gew.%).

VI. Man vermischt 90 Gew.-Teile der Verbindung Nr. 1.010 mit 10 Gew.-Teilen N-Methyl-$\alpha$-pyrrolidon und erhält eine Lösung, die zur Anwendung in Form kleinster Tropfen geeignet ist (Wirkstoffgehalt 90 Gew.%).

VII. 20 Gew.-Teile der Verbindung Nr. 1.001 werden in einer Mischung gelöst, die aus 40 Gew.-Teilen Cyclohexanon, 30 Gew.-Teilen Isobutanol, 20 Gew.-Teilen des Anlagerungsproduktes von 7 Mol Ethylenoxid an 1 Mol Isooctylphenol und 10 Gew.-Teilen des Anlagerungsproduktes von 40 Mol Ethylenoxid an 1 Mol Ricinusöl besteht. Durch Eingießen und feines Verteilen der Lösung in 100 000 Gew.-Teilen Wasser erhält man eine wäßrige Dispersion, die 0,02 Gew.% des Wirkstoffs enthält.

VIII. 20 Gew.-Teile des Wirkstoffs Nr. 1.002 werden mit 3 Gew.-Teilen des Natriumsalzes der Diisobutylnaphthalin-$\alpha$-sulfonsäure, 17 Gew.-Teilen des Natriumsalzes einer Ligninsulfonsäure aus einer Sulfit-Ablauge und 60 Gew.-Teilen pulverförmigem Kieselsäuregel gut vermischt und in einer Hammermühle vermahlen. Durch feines Verteilen der Mischung in 20 000 Gew.-Teilen Wasser erhält man eine Spritzbrühe, die 0,1 Gew.% des Wirkstoffs enthält.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden, wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Wirkstoffkonzentrationen in den anwendungsfertigen Zubereitungen können in größeren Bereichen variiert werden.

Im allgemeinen liegen sie zwischen 0,0001 und 10 %, vorzugsweise zwischen 0,01 und 1 %.

Die Wirkstoffe können auch mit gutem Erfolg im Ultra-Low-Volume-Verfahren (ULV) verwendet werden, wobei es möglich ist, Formulierungen mit mehr als 95 Gew.% Wirkstoff oder sogar den Wirkstoff ohne Zusätze auszubringen.

Die Aufwandmenge an Wirkstoff beträgt unter Freilandbedingungen 0,01 bis 10, vorzugsweise 0,05 bis 5, insbesondere 0,1 bis 1 kg/ha a.S. (aktive Substanz).

Zu den Wirkstoffen können Öle verschiedenen Typs, Herbizide, Fungizide, andere Schädlingsbekämpfungsmittel, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1:10 bis 10:1 zugemischt werden.

Durch Zusatz von anderen Insektiziden bzw. Akariziden läßt sich die Wirkung der erfindungsgemäßen Verbindungen I verbessern und den jeweiligen Erfordernissen anpassen. In dieser Hinsicht geeignete Zusätze sind beispielsweise Phosphorverbindungen, Carbamate, Harnstoffe, Halogenkohlenwasserstoffe sowie Pyrethrum und andere Pyrethroide. Außerdem sind Kombinationen der Verbindungen I mit synergistischen Verbindungen wie

- alpha-(2-(2-Butoxy)ethoxy)-4,5-methylendioxy-2-propyltoluol,
- Propinylethern,
- Propinyloximen,
- Propinylcarbamaten,
- Propinylphosphonaten,
- 2-(3,4-Methylendioxiphenoxy)-3,6,9-trioxaundecan,
- N-(2-Ethylhexyl)bicyclo(2.2.1)hept-5-en-2,3-dicarboximid,
- S,S,S-Tributylphosphortrithioat,
- Octachlorodiisopropylether und
- 1,2-Methylendioxy-4-(2-octylsulfinyl)-propylbenzol vorteilhaft.

Zusätzlich kann auch die Verwendung von Stabilisatoren aus der Gruppe der Antioxidantien und der UV-Absorber vorteilhaft sein.

Synthesebeispiele

Die in den nachstehenden Synthesebeispielen wiedergegebenen Vorschriften wurden unter entsprechender Abwandlung der Ausgangsverbindungen zur Gewinnung weiterer Verbindungen I benutzt. Die so erhaltenen Verbindungen sind in den nachstehenden Tabellen mit physikalischen Angaben aufgeführt.

Herstellung von Ausgangsstoffen

1. 2-Ethyl-6-formyl-2-methyl-2,3-dihydrobenzofuran

In 10 ml absolutem Tetrahydrofuran (THF) werden 0,081 mol Magnesium-Späne vorgelegt und mit einem Tropfen Dibromethan aktiviert. Nach dem Anspringen der Reaktion werden 0,081 mol 6-Brom-2-ethyl-2-methyl-2,3-dihydrobenzofuran gelöst in 70 ml THF (abs.) zugetropft. Es wird 2 h unter Rückfluß gerührt und anschließend 0,089 mol N-Formylpiperidin in 20 ml THF (abs.) bei 0-5 °C zugetropft. Nach 16 h Rühren bei Raumtemperatur werden 80 ml $NH_4Cl$-Lösung (1/2 konz. in $H_2O$) und 50 ml 10 % HCl zugegeben. Nach Zugabe von Diethylether werden die Phasen getrennt. Aus der organischen Phase wurden nach Trocknung, Filtration und Entfernung des Lösungsmittels 0,043 mol Produkt durch Säulenchromatographie erhalten.
$^1$H-NMR (300 MHz; $CDCl_3$)
0,9-1,0 (3H); 1,4 (3H); 1,7-1,8 (2H), 2,9-3,2 (2H); 7,1-7,4 (3H); 9,9 (1H)
Beispiel IV-2 in Tabelle 1

Tabelle 1

6-Formyl-2,3-dihydrobenzofurane IV

| Bsp.-Nr. | $R^2$ | $R^3$ | $^1H$-NMR (300 MHz; $CDCl_3$) |
|---|---|---|---|
| IV-1 | $CH_3$ | $CH_3$ | *1,5 (6H); 3,05 (2H); 7,1-7,4 (3H); 9,9 (1H) |
| IV-2 | $CH_3$ | $CH_2CH3$ | 0,9-1,0 (3H); 1,4 (3H); 1,7-1,8 (2H); 2,9-3,2 (2H); 7,1-7,4 (3H); 9,9 (1H) |
| IV-3 | $CH_2CH_3$ | $CH_2CH_3$ | 0,8-1,05 (6H); 1,6-1,8 (4H) 3,05 (2H); 7,1-7,4 (3H) 9,85 (1H) |

*200 MHz

2. 6-(2,2-Diethyl-2,3-dihydrobenzofuranyl)-methanol

In 80 ml abs. Ethanol wurden 0.0495 mol $NaBH_4$ bei 20 °C vorgelegt. Unter Rühren und ständiger Kühlung werden 0,0985 mol 2,2-Diethyl-6-formyl-2,3-dihydrobenzofuran so zugetropft, daß die Temperatur 25 °C nicht übersteigt. Nach vollständiger Zugabe wird 16 h bei 20 °C gerührt. Das Lösungsmittel wird am Wasserstrahlvakuum entfernt und der verbleibende Rückstand in 200 ml 5 % HCl eingetragen. Die salzsaure Lösung wird mit Ether extrahiert. Die organische Phase wird mit dest. $H_2O$ gewaschen, getrocknet, filtriert und vom Lösungsmittel befreit. Die Ausbeute des gewünschten Produktes beträgt 0,094 mol.
$^1H$-NMR (200 MHz; $CDCl_3$)
0,8-1,0 (6H); 1,6-1,0 (4H); 2,4 (1H); 2,9 (2H); 4,5 (2H); 6,7-7,2 (3H)
Beispiel II-3 in Tabelle 2

3. 1-(2',2'-Dimethyl-2',3'-dihydrobenzofuran-6'-yl)-2-methyl-propan-1-ol

0,18 mol Mg werden mit 10 ml absolutem Diethylether überschichtet. Das Lösungsmittel wird zum Sieden gebracht und es werden 0,18 mol 2-Brompropan gelöst in 100 ml absolutem Diethylether zugetropft. Nach 1 h unter Rückfluß wird auf -20 °C gekühlt. Bei dieser Temperatur werden 0,12 mol 6-Formyl-2,2-dimethyl-2,3-dihydro-benzofuran gelöst in 130 ml absolutem Diethylether zugetropft. Nach 16 h Rühren bei 20 °C wird auf 400 ml Eiswasser gegossen, mit 10 % HCl wird angesäuert und danach mit Diethylether extrahiert. Die organische Phase wird mit Wasser gewaschen. Nach Trocknung und Filtration wird das Lösungsmittel unter vermindertem Druck entfernt. Es werden 0,1 mol Produkt erhalten.
$^1H$-NMR (200 MHz, $CDCl_3$)
0,75 (3H); 0,95 (3H); 1,4 (6H); 1,9 (1H); 2,0 (1H); 2,95 (2H); 4,2 (1H); 6,7-7,1 (3H)
Beispiel II-4 in Tabelle 2.

4. 1-(2'-ethyl-2'-methyl-2',3'-dihydrobenzofuran-6'-yl)-prop-2-in-1-ol

In 50 ml absolutes THF werden bei 0 °C während 25 min Acetylen eingeleitet. Anschließend werden bei -20 bis -25 °C 32 ml einer 1,5n Lösung von Methylmagnesiumchlorid in THF innerhalb von 25 min zugetropft. Bei gleicher Temperatur wird während einer Stunde Acetylen eingeleitet. Anschließend wurde

eine Lösung von 0,032 mol 2-Ethyl-6-formyl-2-methyl-2,3-dihydro-benzofuran in 20 ml absolutem THF zugetropft. Es wurde 2 h bei -20 °C und 16 h bei 20 °C nachgerührt. Die Reaktionslösung wurde in 200 ml Eiswasser gegossen, mit 10 % Salzsäure angesäuert und dnach mit Diethylether extrahiert. Nach Trocknung und Filtration wird das Lösungsmittel unter vermindertem Druck entfernt.

Abschließend erfolgt säulenchromatographische Reinigung an Kieselgel. Es werden 0,023 mol Produkt erhalten.
$^1$H-NMR (300 MHz, $CDCl_3$)
0,9 (3H); 1,4 (3H); 1,7 (2H); 2,6 (2H); 2,95 (1H); 3,05 (1H) 5,35 (1H); 6,9-7,2 (3H)
Beispiel II-7 in Tabelle 2

Tabelle 2

6-(2',3'-Dihydrobenzofuranyl)-methanole II

| Bsp.Nr. | $R^1$ | $R^2$ | $R^3$ | $^1H$-NMR ($CDCl_3$) |
|---|---|---|---|---|
| II-1 | H | $CH_3$ | $CH_3$ | 300 MHz<br>1,45 (6H); 2,6 (1H); 2,95 (2H);<br>4,55 (2H); 6,7-7,1 (3H) |
| II-2 | H | $CH_3$ | $CH_2CH_3$ | 200 MHz<br>0,9 (3H); 1,35 (3H); 1,7 (2H);<br>2,4 (1H); 2,9 (1H); 3,0 (1H);<br>4,55 (2H); 6,85-7,1 (3H) |
| II-3 | H | $CH_2CH_3$ | $CH_2CH_3$ | 200 MHz<br>0,8-1,0 (6H); 1,6-1,9 (4H);<br>2,4 (1H); 2,9 (2H); 4,5 (2H);<br>6,7-7,2 (3H) |
| II-4 | $CH(CH_3)_2$ | $CH_3$ | $CH_3$ | 200 MHz<br>0,75 (3H); 0,95 (3H); 1,4 (6H);<br>1,9 (1H); 2,0 (1H); 2,95 (2H);<br>4,2 (1H); 6,7-7,1 (3H) |
| II-5 | $CH(CH_3)_2$ | $CH_3$ | $CH_2CH_3$ | 300 MHz<br>0,75 (3H); 0,9-1,0 (6H); 1,4 (3H),<br>1,6-2,0 (4H); 2,85 (1H); 3,05 (1H);<br>4,2 (1,4); 6,6-7,1 (3H) |
| II-6 | C≡CH | $CH_3$ | $CH_3$ | 250 MHz<br>1,45 (6H); 2,55 (1H); 2,60 (1H);<br>3,0 (2H); 5,35 (1H); 6,9-7,2 (3H) |
| II-7 | C≡CH | $CH_3$ | $CH_2CH_3$ | 300 MHz<br>0,9 (3H); 1,4 (3H); 1,7 (2H);<br>2,6 (2H); 2,95 (1H); 3,05 (1H);<br>5,35 (1H); 6,8-7,2 (3H) |

5. 3-(2'-Chlor-3',3',3'-trifluoro-prop-2'-en-yl)-2,2-dimethyl-cyclopropan-1-carbonsäure-[(2'-ethyl-2'-methyl-2',3'-dihydrobenzo-furan-6'-yl)-α-cyano]-methylester

In 75 ml Diethylether werden 0,016 mol 2-Ethyl-6-formyl-2-methyl-2,3-dihydrobenzofuran gelöst und mit einer Lösung von 0,018 mol KCN in 10 ml $H_2O$ versetzt. Nach Zugabe von 0,3 ml Phasentransfer-Katalysator (Lutensit®), 0,3 ml $NaHSO_3$-Lösung und 0,017 ml 3-(2'-Chlor-3',3',3'-trifluoro-prop-2'-enyl)-2,2-dimethyl-cyclopropan-1-carbonsäure-chlorid wird 4 Tage bei 20 °C gerührt. Die Phasen werden getrennt

und die organische Phase 2 mal mit $H_2O$ gewaschen. Nach Trocknung, Filtration, Entfernen des Lösungsmittels und Chromatographie an Kieselgel werden 0,003 ml Produkt isoliert.
$^1$H-NMR: (200 MHz, $CDCl_3$)
1,2-1,4 (6H); 1,65 (6H); 1,8-2,5 (2H); 3,0 (2H); 6,05-6,4 (1,5H); 6,8-7,3 (3,5H)
Beispiel 1.008

6. 3-(2'-Chlor-3',3',3'-trifluoro-prop-2'-enyl)-2,2-dimethyl-cyclopropan-1-carbonsäure-(2',2'-diethyl-2',3'-dihydrobenzofuran-6'-yl)-methyleester

In 20 ml Toluol werden 0,015 mol 6-(2,2-Diethyl-2,3-dihydrobenzofuranyl)-methanol gelöst und mit 0,016 mol Picolin versetzt. Zu diesem Gemisch gibt man 0,016 mol 3-(2'-Chlor-3',3',3'-trifluoro-prop-2-enyl)-2,2-dimethyl-cyclopropan-1-carbonsäure-chlorid. Nach 16 h wird über eine Kieselgelnutsche filtriert. Das Eluat wird vom Lösungsmittel befreit und bei 60 °C an der Ölpumpe getrocknet. Es werden 0,012 mol Produkt erhalten.
$^1$H-NMR (200 MHz, $CDCl_3$)
0,9 (6H); 1,05-1,4 (6H); 1,7 (4H); 1,8-2,5 (2H); 2,95 (2H); 5,05 (2H); 6,15 (0,5H); 6,7-7,2 (3,5H);
Beispiel 1.003

Wirkstofftabelle 3

| Bsp.Nr. | $R^1$ | $R^2$ | $R^3$ | MHz | $^1H$-NMR ($CDCl_3$) [δ] ppm |
|---|---|---|---|---|---|
| 1.001* | H | $CH_3$ | $CH_3$ | 300 | 1,3 (6H); 1,5 (6H); 2,0-2,2 (2H); 3,0 (2H); 5,1 (2H); 6,7-7,2 (4H) |
| 1.002 | H | $CH_3$ | $CH_2CH_3$ | 300 | 0,95 (3H); 1,2 (3H); 1,25-1,45 (6H); 1,7 (2H); 1,8-2,05 (1H); 2,15-2,45 (1H); 2,9 (1H); 3,1 (1H); 5,1 (2H); 6,1 (0,5H); 6,6-7,2 (3,5H) |
| 1.003 | H | $CH_2CH_3$ | $CH_2CH_3$ | 200 | 0,9 (6H); 1,05-1,4 (6H); 1,7 (4H); 1,8-2,5 (2H); 2,95 (2H); 5,05 (2H); 6,15 (0,5H); 6,7-7,2 (3,5H) |
| 1.004 | $CH(CH_3)_2$ | $CH_3$ | $CH_3$ | 200 | 0,8 (3H); 0,9 (3H); 1,1-1,3 (6H); 1,6 (6H); 1,8-2,4 (3H); 2,95 (2H); 5,35 (1H); 6,1 (0,5H); |
| 1.005 | $CH(CH_3)_2$ | $CH_3$ | $CH_2CH_3$ | 300 | 0,7-1,0 (9H); 1,05-1,35 (6H); 1,4 (3H); 1,65-2,45 (5H); 2,85 (1H); 3,05 (1H); 5,4 (1H); 6,15 (0,5H); 6,5-7,2 (3,5H) |
| 1.006 | C≡CH | $CH_3$ | $CH_3$ | 300 | 1,0-1,4 (6H); 1,45 (6H); 1,7-2,5 (2H); 2,65 (1H); 3,0 (2H); 6,15 (0,5H); 6,35 (1H); 6,8-7,2 (3,5H) |
| 1.007** | C≡CH | $CH_3$ | $CH_2CH_3$ | 300 | 0,95 (3H); 1,2-1,4 (6H); 1,45 (3H); 1,75 (2H); 1,9-2,2 (2H); 2,65 (1H); 2,9 (1H); 3,1 (1H); 6,35 (1H); 6,8-7,2 (4H) |

Wirkstofftabelle 3

| Bsp.Nr. | $R^1$ | $R^2$ | $R^3$ | MHz | $^1H$-NMR ($CDCl_3$) [δ] ppm |
|---|---|---|---|---|---|
| 1.008 | C≡N | $CH_3$ | $CH_3$ | 200 | 1,2-1,4 (6H); 1,65 (6H); 1,8-2,5 (2H); 3,0 (2H); 6,05-6,4 (1,5H); 6,8-7,3 (3,5H) |
| 1.009 | C≡N | $CH_3$ | $CH_2CH_3$ | 300 | 0,95 (3H); 1,1-1,4 (6H); 1,45 (3H); 1,75 (2H); 1,8-2,5 (2H); 2,95 (1H); 3,1 (1H); 6,1-6,4 (1,5H); 6,8-7,3 (3,5H) |
| 1.010*** | H | $CH_3$ | $CH_3$ | 300 | 1,25 (3H); 1,35 (3H); 1,47 (6H); 1,8 (1H); 2,42 (1H); 3,0 (2H); 5,1 (2H); 6,15 (1H); 6,75-7,15 (3H) |

* Isomerenverhältnis bezüglich der Substituenten am Cyclopropanring 95 : 5 (cis : trans)

** cis-Isomer bezüglich der Substituenten am Cyclopropanring

*** trans-Isomer bezüglich der Substituenten am Cyclopropanring

Anwendungsbeispiele:

Die insektizide bzw. akarizide Wirkung der Verbindungen der allgemeinen Formel I ließ sich durch folgende Versuche zeigen:

Die Wirkstoffe wurden

a) als 0,1%ige Lösung in Aceton oder
b) als 10%ige Emulsion in einem Gemisch aus 70 Gew.-% Cyclohexanol, 20 Gew.-% Nekanil® LN (Lutensol® AP6, Netzmittel mit Emulgier- und Dispergierwirkung auf der Basis ethoxylierter Alkylphenole) und 10 Gew.-% Emulphor® EL (Emulan® EL, Emulgator auf der Basis ethoxylierter Fettalkohole)

aufbereitet und entsprechend der gewünschten Konzentration mit Aceton im Fall von a) bzw. mit Wasser im Fall von b) verdünnt.

Nach Abschluß der Versuche wurde die jeweils niedrigste Konzentration ermittelt, bei der die Verbindungen im Vergleich zu unbehandelten Kontrollversuchen noch eine 80 - 100%ige Hemmung bzw. Mortalität hervorriefen (Wirkschwelle bzw. Minimalkonzentration (mg)).

A) Aphis fabae (Schwarze Laus); Kontaktwirkung, Spritzversuch

Junge Bohnenpflanzen (Vicia faba) mit einer starken Kolonie der Schwarzen Laus werden mit der

wäßrigen Aufbereitung des Versuchsmittels behandelt. Die Pflanze kommt dazu auf den Drehteller der Spritzkabine und wird mit insgesamt 50 ml der Aufbereitung allseitig besprüht.

Nach 24 Stunden erfolgt die Versuchsauswertung durch Bestimmung der Mortalitätsrate.

Die Wirkschwellen der Verbindungen 1.001, 1.002 und 1.010 lagen bei 400 ppm bzw. 1000 ppm.

B) Heliothis virescens, ovo-larvizide Wirkung

Blattstanzstücke (Ø 22 mm) von Buschbohnen werden in die wäßrige Wirkstoffaufbereitung getaucht. Das Blattstück wird mit der Oberseite auf einen feuchten Filter in eine Kunststoffpetrischale (Ø 60 mm) gelegt und mit ca. 15 Heliothis-Eiern belegt. Die Eier sind nicht älter als 24 Stunden. Man schließt die Petrischale und beurteilt nach 4 Tagen Schlupf und Mortalität der Jungraupen.

Die Wirkschwellen der Verbindungen 1.001 und 1.010 lagen bei 100 ppm.

C) Plutella maculipennis (Kohlschabe), fraßverhindernde Wirkung

Junge Kohlrabiblätter werden 3 sec. in die wäßrige Aufbereitung der Prüfsubstanz getaucht und auf einen mit 0,5 ml Wasser angefeuchteten Rundfilter (Ø 9 cm) in eine Petrischale gelegt (Ø 10 cm). Darauf wird das Blatt mit 10 Raupen im 4. Larvenstadium besetzt und die Petrischale geschlossen. Nach 48 Stunden beurteilt man die Fraßverhinderung in Prozent.

Die Wirkschwellen der Verbindungen 1.001, 1.002 und 1.010 lagen bei 10 ppm bzw. 20 ppm.

D) Plutella maculipennis (Kohlschaben), Kontaktwirkung

Blätter von jungen Kohlpflanzen werden 3 Sekunden in die wäßrige Wirkstoffemulsion getaucht und nach kurzem Abtropfen auf einen angefeuchteten Filter in eine Petrischale gelegt. Das Blatt wird darauf mit 10 Raupen des 4. Stadiums belegt.

Nach 48 Stunden beurteilt man die Mortalität in Prozent.

Die Wirkschwellen der Verbindungen 1.001, 1.002 und 1.010 lagen bei 4 bis 20 ppm.

E) Tetranychus telarius (Rote Spinne); Kontaktwirkung

Getopfte Buschbohnen, die das zweite Folgeblattpaar zeigen, und einen starken Milbenbefall aufweisen, werden in der Spritzkabine mit der wäßrigen Wirkstoffaufbereitung tropfnaß gespritzt. Die Pflanzen kommen dazu auf den Drehteller und werden von allen Seiten mit insgesamt 50 ml Spritzbrühe besprüht.

Die Wirkung wird nach 5 Tagen mittels Binokular festgestellt. Die Pflanzen stehen während dieser Zeit unter normalen Gewächshausbedingungen.

Die Wirkschwellen der Verbindungen 1.001 und 1.002 lagen bei 1000 ppm bzw. 200 ppm.

## Patentansprüche

**1.** 2,3-Dihydrobenzofurylmethylester der allgemeinen Formel I,

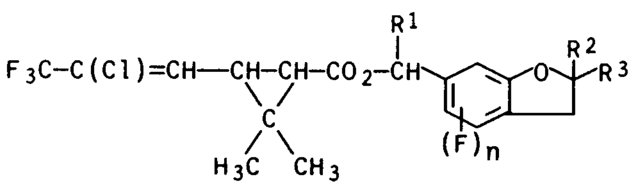

I

in der die Substituenten und der Index folgende Bedeutung haben:

| | |
|---|---|
| $R^1$ | Wasserstoff, $C_1$-$C_4$-Alkyl, $C_2$-$C_4$-Alkenyl, $C_2$-$C_4$-Alkinyl oder Cyano; |
| $R^2$ und $R^3$ | unabhängig voneinander Wasserstoff oder $C_1$-$C_4$-Alkyl und |
| n | 0 oder 1. |

**2.** 2,3-Dihydrobenzofurylmethylester der allgemeinen Formel I gemäß Anspruch 1, in denen

| | |
|---|---|
| $R^1$ | Wasserstoff, $C_1$-$C_4$-Alkyl, $C_2$-$C_4$-Alkinyl oder Cyano, |
| $R^2$ und $R^3$ | unabhängig voneinander Wasserstoff oder $C_1$-$C_4$-Alkyl und |

n 0
bedeuten.

**3.** 2,3-Dihydrobenzofurylmethylester der allgemeinen Formel I gemäß Anspruch 1, in denen
$R^1$ Wasserstoff, $C_1$-$C_4$-Alkyl, $C_2$-$C_4$-Alkenyl, $C_2$-$C_4$-Alkinyl oder Cyano,
$R^2$ und $R^3$ unabhängig voneinander $C_1$-$C_4$-Alkyl und
n 0
bedeuten.

**4.** Verfahren zur Herstellung der Verbindungen I gemäß Anspruch 1 dadurch gekennzeichnet, daß man ein 2,3-Dihydrobenzofurylmethylderivat der allgemeinen Formel II

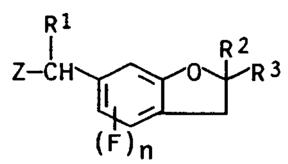

II

in der Z für Hydroxy oder Halogen steht, in an sich bekannter Weise mit einer Carbonsäure der allgemeinen Formel IIIa

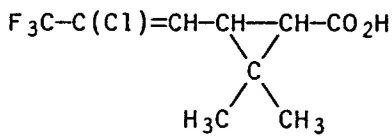

IIIa

oder deren Derivaten verestert.

**5.** Verfahren zur Herstellung der Verbindungen I gemäß Anspruch 1, in denen $R^1$ Cyano bedeutet, dadurch gekennzeichnet, daß man einen 2,3-Dihydrobenzofurylaldehyd der allgemeinen Formel IV

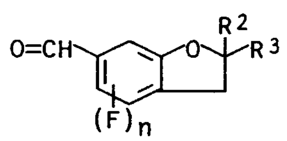

IV

in an sich bekannter Weise in Gegenwart eines Alkalimetall- oder eines Erdalkalimetallcyanids mit dem Halogenid einer Carbonsäure der allgemeinen Formel IIIb

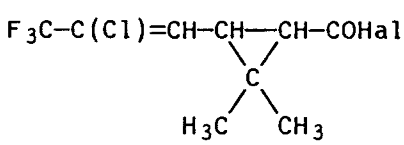

IIIb

in der Hal für ein Halogenatom steht, umsetzt.

**6.** Verfahren zur Herstellung der Verbindungen IV gemäß Anspruch 5, dadurch gekennzeichnet, daß man ein 2,3-Dihydrobenzofurylhalogenid der allgemeinen Formel V

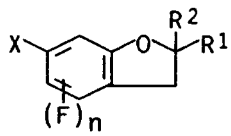

V

in der X Halogen bedeutet, in an sich bekannter Weise metalliert und anschließend ebenfalls in an sich bekannter Weise formyliert.

**7.** Verfahren zur Herstellung der Verbindungen II gemäß Anspruch 4, in denen Z für Hydroxy steht, dadurch gekennzeichnet, daß man eine Verbindung der allgemeinen Formel IV in an sich bekannter Weise reduziert oder mit einer metallorganischen Verbindung $MR^1$, in der M für ein Alkalimetall, ein Erdalkalimetall oder ein Übergangsmetall steht und $R^1$ nicht Wasserstoff bedeutet, in an sich bekannter Weise umsetzt.

**8.** Verfahren zur Herstellung der Verbindungen II gemäß Anspruch 4, in denen Z für Halogen steht, dadurch gekennzeichnet, daß man eine Verbindung der allgemeinen Formel II gemäß Anspruch 4, in der Z für Hydroxy steht, in an sich bekannter Weise mit einem Halogenierungsmittel umsetzt.

**9.** Mittel zur Bekämpfung von Schädlingen, enthaltend eine Verbindung der allgemeinen Formel I gemäß Anspruch 1 und inerte Zusatzstoffe.

**10.** Verfahren zur Bekämpfung von Schädlingen, dadurch gekennzeichnet, daß man die Schädlinge und/oder deren Lebensraum mit einer wirksamen Menge einer Verbindung der allgemeinen Formel I gemäß Anspruch 1 behandelt.

Europäisches Patentamt

# EUROPÄISCHER RECHERCHENBERICHT

Nummer der Anmeldung

EP 91113881.6

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl.⁵) |
| D,A | DE - C3 - 2 108 932 (BASF) * Ansprüche 1,2 * -- | 1,9 | C 07 D 307/80<br>A 01 N 43/12 |
| D,A | EP - B1 - 0 023 637 (BASF) * Ansprüche 1-5 * -- | 1,9 | |
| A | EP - A1 - 0 238 445 (CIBA-GEIGY) * Ansprüche 1,8 * -- | 1,9 | |
| A | FR - A1 - 1 511 331 (FMC) * Anspruch 1 * -- | 1 | |
| A | CHEMICAL ABSTRACTS, Band 71, Nr. 19, 10. November 1969, Columbus, Ohio, USA BROOKER, PETER J. et al. "Benzofuranyl carbamates as pesticides." Seite 364, Spalte 2, Zusammenfassung-Nr. 91 281x & S. African 67 04,114 ---- | 1 | RECHERCHIERTE SACHGEBIETE (Int. Cl.⁵)<br>C 07 D 307/00 |

Der vorliegende Recherchenbericht wurde für alle Patentansprüche erstellt.

| Recherchenort | Abschlußdatum der Recherche | Prüfer |
|---|---|---|
| WIEN | 04-11-1991 | HAMMER |

KATEGORIE DER GENANNTEN DOKUMENTEN
- X : von besonderer Bedeutung allein betrachtet
- Y : von besonderer Bedeutung in Verbindung mit einer anderen Veröffentlichung derselben Kategorie
- A : technologischer Hintergrund
- O : nichtschriftliche Offenbarung
- P : Zwischenliteratur
- T : der Erfindung zugrunde liegende Theorien oder Grundsätze
- E : älteres Patentdokument, das jedoch erst am oder nach dem Anmeldedatum veröffentlicht worden ist
- D : in der Anmeldung angeführtes Dokument
- L : aus andern Gründen angeführtes Dokument
- & : Mitglied der gleichen Patentfamilie, übereinstimmendes Dokument

EPA Form 1503 03 82